(19) Europäisches Patentamt
European Patent Office
Office européen des brevets

(11) EP 0 982 308 B1

(12) **FASCICULE DE BREVET EUROPEEN**

(45) Date de publication et mention de la délivrance du brevet:
**26.02.2003 Bulletin 2003/09**

(51) Int Cl.[7]: **C07D 495/14**, A61K 31/40 // (C07D495/14, 333:00, 209:00, 209:00)

(21) Numéro de dépôt: **99401892.7**

(22) Date de dépôt: **26.07.1999**

(54) **Dérivés de 8H-(2,3-b)-pyrrolizine-8-one, leur procédé de préparation et les compositions pharmaceutiques qui les contiennent**

8H-(2,3-b)-Pyrrolozin-8-one Derivate, Verfahren zur ihre Herstellung und sie enthaltende pharmazeutische Zusammensetzungen

Derivatives of the 8H-(2,3-b)-pyrrolizine-8-one, process for their preparation and pharmaceutical compositions containing them

(84) Etats contractants désignés:
**AT BE CH CY DE DK ES FI FR GB GR IE IT LI LU MC NL PT SE**

(30) Priorité: **27.07.1998 FR 9809552**

(43) Date de publication de la demande:
**01.03.2000 Bulletin 2000/09**

(73) Titulaire: **LES LABORATOIRES SERVIER**
**92200 Neuilly sur Seine (FR)**

(72) Inventeurs:
- **Rault, Sylvain**
  **14370 Moult (FR)**
- **Enguehard, Cécile**
  **14000 Caen (FR)**
- **Lancelot, Jean-Charles**
  **14400 Le Bourg (FR)**
- **Robba, Max**
  **75004 Paris (FR)**
- **Atassi, Ghanem**
  **92210 Saint Cloud (FR)**
- **Pierre, Alain**
  **78580 Les Alluets le Roi (FR)**
- **Caignard, Daniel-Henri**
  **78230 Le Pecq (FR)**
- **Renard, Pierre**
  **78150 Le Chesnay (FR)**

(56) Documents cités:
**EP-A- 0 341 213** **EP-A- 0 718 299**

- **J.-C. LANCELOT ET AL.: JOURNAL OF HETEROCYCLIC CHEMISTRY, vol. 31, 1994, pages 501-504, XP002097875 PROVO US**
- **DATABASE WPI Week 9537 Derwent Publications Ltd., London, GB; AN 95-280907 XP002097876 & JP 07 179471 A (YOSHITOMI PHARM. IND. K.K.), 18 juillet 1995 (1995-07-18)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 982 308 B1

Printed by Jouve, 75001 PARIS (FR)

## Description

La présente invention concerne de nouveaux dérivés de 8*H*-thiéno-[2,3-*b*]pyrrolizine-8-one, leur procédé de préparation et les compositions pharmaceutiques qui les contiennent.

Les besoins de la thérapeutique anticancéreuse exigent le développement constant de nouveaux agents antitumoraux, dans le but d'obtenir des médicaments à la fois plus actifs et mieux tolérés. Plus particulièrement, les tumeurs solides posent un problème majeur à la chimiothérapie anticancéreuse, de par leur résistance, intrinsèque et/ou acquise, aux produits existants.

Les composés de l'invention, outre le fait qu'ils soient nouveaux, présentent une activité in-vivo et in-vitro surprenante et particulière. Ainsi, les composés découverts par la Demanderesse possèdent des propriétés antitumorales qui les rendent particulièrement utiles pour le traitement des cancers, et de façon avantageuse pour le traitement des tumeurs solides.

Des composés de structures proches ont été décrits dans la littérature, notamment dans la demande de brevet EP 718299, ces composés servant d'intermédiaires de synthèse pour l'obtention d'éthers d'oximes tricycliques. Aucune activité thérapeutique n'est décrite pour ces intermédiaires de synthèse, alors que les produits finaux, c'est-à-dire les éthers d'oximes tricycliques, possèdent une très forte affinité pour les récepteurs $5HT_{2C}$ et/ou $5HT_3$, domaine thérapeutique différent de celui de la présente invention.

Plus spécifiquement, la présente invention concerne les composés de formule (I) :

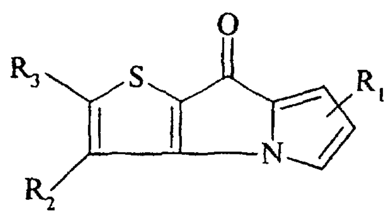

(I)

dans laquelle :

- $R_1$ représente un atome d'hydrogène, d'halogène, un groupement alkyle ($C_1$-$C_6$) linéaire ou ramifié, nitro, hydroxy, alkoxy ($C_1$-$C_6$) linéaire ou ramifié, trihalogénoalkyle ($C_1$-$C_6$) linéaire ou ramifié, trihalogénoalkoxy ($C_1$-$C_6$) linéaire ou ramifié, ou un groupement amino (éventuellement substitué par un ou deux groupements, identiques ou différents, indépendamment l'un de l'autre, alkyle ($C_1$-$C_6$) linéaire ou ramifié),

- $R_2$ représente un groupement aryle éventuellement substitué ou hétéroaryle éventuellement substitué,

- $R_3$ représente un atome d'hydrogène, d'halogène, un groupement alkyle ($C_1$-$C_6$) linéaire ou ramifié, nitro, hydroxy, alkoxy ($C_1$-$C_6$) linéaire ou ramifié, trihalogénoalkyle ($C_1$-$C_6$) linéaire ou ramifié, trihalogénoalkoxy ($C_1$-$C_6$) linéaire ou ramifié, ou un groupement amino (éventuellement substitué par un ou deux groupements, identiques ou différents, indépendamment l'un de l'autre, alkyle ($C_1$-$C_6$) linéaire ou ramifié),

leurs isomères ainsi que leurs sels d'addition à un acide ou à une base pharmaceutiquement acceptable,
étant entendu que lorsque $R_1$ et $R_3$ représentent simultanément un atome d'hydrogène, alors $R_2$ ne peut pas représenter un groupement phényle éventuellement substitué en position *para* par un atome de brome, de chlore, de fluor, un groupement méthoxy ou hydroxy.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Par groupement aryle, on comprend un groupement phényle, naphtyle, dihydronaphtyle, tétrahydronaphtyle, indényle, indanyle ; par groupement hétéroaryle, on comprend un groupement aromatique mono ou bicyclique, de 5 à 12 chaînons contenant un, deux ou trois hétéroatomes, identiques ou différents, choisis parmi oxygène, azote ou soufre. Parmi ces groupements hétéroaryle, on peut citer, à titre non limitatif, le groupement thiényle, furyle, pyrrolyle, pyridyle ou indolyle ; par éventuellement substitué, on comprend un groupement aryle ou hétéroaryle tel que défini précédemment substitué par un ou plusieurs groupements, identiques ou différents, indépendamment l'un de l'autre, choisis parmi halogène, alkyle ($C_1$-$C_6$) linéaire ou ramifié, hydroxy, alkoxy ($C_1$-$C_6$) linéaire ou ramifié, trihalogénoalkoxy

($C_1$-$C_6$) linéaire ou ramifié, nitro, cyano, amino, alkylamino ($C_1$-$C_6$) linéaire ou ramifié, dialkylamino ($C_1$-$C_6$) linéaire ou ramifié, acyle ($C_1$-$C_6$) linéaire ou ramifié, aminocarbonyle, (la partie amino étant éventuellement substituée par un ou deux groupements, identiques ou différents, alkyle ($C_1$-$C_6$) linéaire ou ramifié), carboxy, alkoxycarbonyle ($C_1$-$C_6$) linéaire ou ramifié, méthylènedioxy, éthylénedioxy, alkylcarbonyloxy ($C_1$-$C_6$) linéaire ou ramifié, et arylalkoxy ($C_1$-$C_6$) linéaire ou ramifié.

Le substituant $R_2$ préféré selon l'invention est le substituant phényle substitué par au moins un groupement tel que défini précédemment.

Selon une variante avantageuse de l'invention, le substituant $R_2$ préféré est le substituant phényle substitué par au moins un groupement choisi parmi hydroxy, alkoxy ($C_1$-$C_6$) linéaire ou ramifié et alkylcarbonyloxy ($C_1$-$C_6$) linéaire ou ramifié.

Les substituants $R_1$ et $R_3$ préférés selon l'invention sont l'atome d'hydrogène.

Les composés préférés selon l'invention sont le 3-(3,4-dihydroxyphényl)-8*H*-thiéno [2,3-*b*]pyrrolizine-8-one, et l'acétate de 2-méthoxy-5-(8-oxo-8*H*-thiéno[2,3-b]pyrrolizin-3-yl)phényl.

Les isomères, ainsi que les sels d'addition à un acide ou à une base pharmaceutiquement acceptable des composés préférés font partie intégrante de l'invention.

L'invention s'étend également au procédé de préparation des composés de formule (I), caractérisé en ce qu'on utilise comme produit de départ un composé de formule (II) :

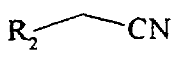

(II)

dans laquelle $R_2$ est tel que défini dans la formule (I), composés de formule (II) que l'on fait réagir en présence d'un métal alcalin, avec du formiate d'éthyle, pour conduire aux composés de formule (III) :

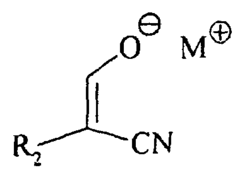

(III)

dans laquelle $R_2$ est tel que défini précédemment et $M^+$ représente un cation alcalin, tel que le cation sodium ou potassium,
composés de formule (III) que l'on place dans des conditions d'addition nucléophile en présence de chlorure de phénylsulfonyle, pour conduire aux composés de formule (IV) :

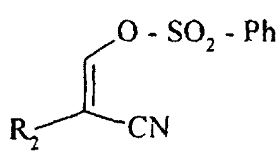

(IV)

dans laquelle $R_2$ est tel que défini précédemment,
composés de formule (IV) sur lequel on fait réagir du thioglycolate de méthyle, pour conduire aux composés de formule (V) :

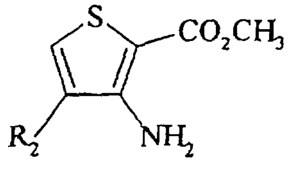

(V)

dans laquelle $R_2$ est tel que défini précédemment,

composés de formule (V) dont on protège le groupement amino, selon des méthodes classiques de synthèse organique, puis que l'on fait réagir avec un composé de formule (VI) :

$$R_3 - X \qquad (VI)$$

dans laquelle X représente un groupement libérable et $R_3$ est tel que défini dans la formule (I), puis dont on déprotège la fonction amino, pour conduire aux composés de formule (VII) :

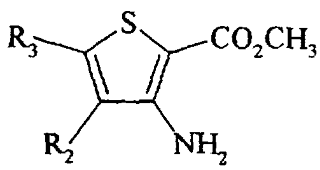

(VII)

dans laquelle $R_2$ et $R_3$ sont tels que définis dans la formule (I), composés de formule (VII) que l'on place en présence de diméthoxytétrahydrofurane et de chlorure de 4-chloropyridinium, pour conduire aux composés de formule (VIII) :

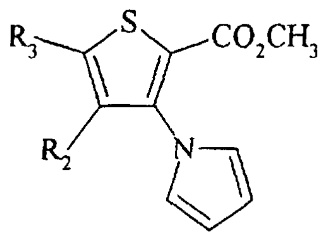

(VIII)

dans laquelle $R_2$ et $R_3$ sont tels que définis précédemment,
puis que l'on fait réagir en présence de pyrrolidine, pour conduire aux composés de formule (IX) :

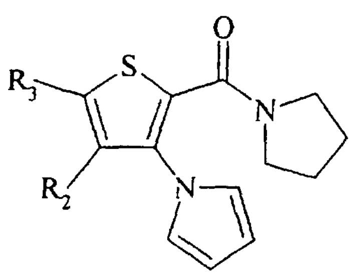

(IX)

dans laquelle $R_2$ et $R_3$ sont tels que définis précédemment,
composés de formule (IX) que l'on cyclise avec de l'oxychlorure de phosphore, pour conduire aux composés de formule (I/a), cas particulier des composés de formule (I) :

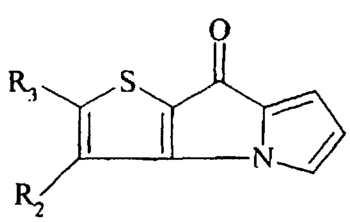

(I/a)

dans laquelle $R_2$ et $R_3$ sont tels que définis précédemment,
composés de formule (I/a) dont on fonctionnalise le noyau pyrrole selon des conditions classiques de synthèse organique, pour conduire aux composés de formule (I/b), cas particulier des composés de formule (I) :

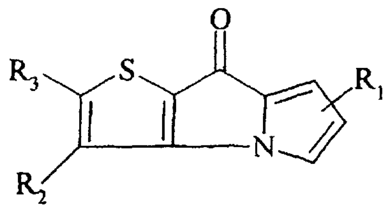

(I/b)

dans laquelle $R_2$, $R_3$ et $R_1$ sont tels que définis dans la formule (I), excepté que $R_1$ ne peut pas représenter un atome d'hydrogène, les composés (I/a) et (I/b) formant l'ensemble des composés de l'invention, que l'on purifie, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

Les composés de formules (II) et (VI) sont soit des composés commerciaux, soit obtenus selon des méthodes classiques de synthèse organique.

Les composés de formule (I) présentent des activités antitumorales particulièrement intéressantes. Ils ont une excellente cytotoxicité in vitro sur des lignées cellulaires et notamment des lignées établies à partir de tumeurs solides humaines, et sont très bien tolérés in vivo. Les propriétés caractéristiques de ces composés permettent leur utilisation en thérapeutique en tant qu'agents antitumoraux.

La présente invention a également pour objet les compositions pharmaceutiques contenant les produits de formule (I), leurs isomères optiques ou un de leurs sels d'addition à une base ou un acide pharmaceutiquement acceptable, seul ou en combinaison avec un ou plusieurs excipients ou véhicules inertes, non toxiques, pharmaceutiquement acceptable.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les gouttes oculaires ou nasales, etc...

La posologie utile varie selon l'âge et le poids du patient, la voie d'administration, la nature et la sévérité de l'affection et la prise de traitements éventuels associés et s'échelonne de 1 mg à 500 mg en une ou plusieurs prises par jour.

Les exemples suivants illustrent l'invention mais ne la limitent en aucune façon. Les produits de départ utilisés sont des produits connus ou préparés selon des modes opératoires connus.

Les différents stades conduisent à des intermédiaires de synthèse, utiles pour la préparation des composés de l'invention.

Les structures des composés décrits dans les exemples et les stades de synthèse ont été déterminées selon les techniques spectrophotométriques usuelles (infrarouge, résonance magnétique nucléaire, spectrométrie de masse ...).

**EXEMPLE 1 : 3-(2-Méthoxyphényl)-8*H*-thiéno[2,3-*b*]pyrrolizine-8-one**

***Stade A :*** *α-Hydroxyméthylidène-(2-méthoxyphényl)acétonitrile, sel de sodium*

A une solution de 0,34 mol de sodium dans 150 ml de méthanol, refroidie à 0°C, sont additionnés successivement 1 équivalent de formiate d'éthyle, et 1 équivalent de 2-(2-méthoxyphényl)acétonitrile. Le milieu réactionnel est alors chauffé pendant une heure à 60°C, puis dilué par addition de solvant, laissant apparaître un nouveau précipité qui est filtré puis séché sous vide permettant d'isoler le produit attendu.
*Point de fusion : > 250°C*

***Stade B :*** *α-(Phénylsulfonyloxyméthylidène)-2-méthoxyphénylacétonitrile*

A une suspension de 0,3 mol du composé obtenu dans le stade A dans 100 ml de diméthylformamide, refroidie à 0°C est additionné, goutte à goutte, 1 équivalent de chlorure de 2-méthoxyphényl-sulfonyle. Après 2 heures d'agitation, 100 ml d'eau sont additionnés entraînant la formation d'un précipité. Celui-ci est filtré puis séché sous vide

permettant d'isoler le produit attendu.
*Point de fusion :* > 250°C

***Stade C* : 3-Amino-4-(2-méthoxyphényl)-2-thiophènecarboxylate de méthyle**

11 mmol de sodium sont dissous dans 100 ml de méthanol. Après refroidissement du milieu réactionnel à l'aide d'un bain de glace, 4,5 mmol de thioglycolate de méthyle, puis 3,7 mmol du composé obtenu dans le stade B sont additionnés. Après 2 heures de réaction, 100 ml d'eau sont additionnés, entraînant la formation d'un précipité qui est filtré puis séché, permettant d'isoler le produit attendu.
*Point de fusion : 110°C*

***Stade D : 4-(2-Méthoxyphényl)-3-(1H-1-pyrrolyl)-2-thiophènecarboxylate de méthyle***

5,2 mmol de diméthoxytétrahydrofurane et 5,2 mmol de chlorure de 4-chloropyridinium dans 100 ml de dioxane, sont agités 15 minutes à température ambiante, puis 5,2 mmol du composé obtenu dans le stade C sont additionnés. Le mélange réactionnel est alors porté au reflux du solvant pendant 3 heures, puis filtré. Après concentration sous pression réduite du filtrat, le résidu obtenu est cristallisé par trituration dans l'éther éthylique, permettant d'isoler le produit attendu.
*Point de fusion : 148°C*

***Stade E : 4-(2-Méthoxyphényl)-3-(1H-1-pyrrolyl)-2-thiophène-N-pyrrolidino carboxamide***

Une solution de 3,8 mmol du composé obtenu dans le stade D, dans 50 ml de pyrrolidine est portée au reflux pendant 3 heures. Après refroidissement, 100 ml d'eau sont ajoutés entraînant la formation d'un précipité qui est filtré, rincé à l'éther de pétrole, puis séché sous vide, permettant d'isoler le produit attendu.
*Point de fusion : 108°C*

***Stade F : 3-(2-Méthoxyphényl)-8H-thiéno[2,3-b]pyrrolizine-8-one***

Une solution de 2,7 mmol du composé obtenu dans le stade E dans 50 ml d'oxychlorure de phosphore est portée au reflux pendant 3 heures. Après refroidissement et évaporation du solvant sous pression réduite, le résidu est cristallisé dans l'éther, filtré et lavé à l'éther de pétrole. Les cristaux sont alors jetés dans 100 ml de soude aqueuse à 10 % et agités à 50°C pendant 1 heure. Le solide est ensuite filtré, séché puis purifié par chromatographie sur gel de silice (chloroforme) permettant d'isoler le produit attendu.
*Point de fusion : 142°C*

| *Microanalyse élémentaire :* | | | |
|---|---|---|---|
| | *% C* | *% H* | *% N* |
| *calculé* | *68,31* | *3,94* | *4,98* |
| *trouvé* | *68,24* | *4,04* | *4,84* |

**EXEMPLE 2 :3-(3-Méthoxyphényl)-8*H*-thiéno-(2,3-*b*]pyrrolizine-8-one**

On procède comme dans l'exemple 1, des stades A à F, en utilisant comme substrat dans le stade A le 2-(3-méthoxyphényl)acétonitrile.
*Point de fusion :* 150°C

| *Microanalyse élémentaire :* | | | |
|---|---|---|---|
| | *% C* | *% H* | *% N* |
| *calculé* | *68,31* | *3,94* | *4,98* |
| *trouvé* | *63,48* | *3,94* | *4,29* |

**EXEMPLE 3 : 3-(3,43-(3,4-Diméthoxyphényl)-8*H*-thiéno-[2,3-*b*]pyrrolizine-8-one**

On procède comme dans l'exemple 1, des stades A à F, en utilisant comme substrat dans le stade A le

2-(3,4-diméthoxyphényl)-acétonitrile.
*Point de fusion : 190°C*

| *Microanalyse élémentaire :* | | | |
|---|---|---|---|
| | *% C* | *% H* | *% N* |
| *calculé* | *65,58* | *4,21* | *4,50* |
| *trouvé* | *64,45* | *4,12* | *4,46* |

**EXEMPLE 4 : 3-(3,4-Méthylènedioxyphényl)-8*H*-thiéno-[2,3-*b*]pyrrolizine-8-one**

On procède comme dans l'exemple 1, des stades A à F, en utilisant comme substrat dans le stade A, celui utilisé dans l'exemple 3.
*Point de fusion : 168°C*

| *Microanalyse élémentaire :* | | | |
|---|---|---|---|
| | *% C* | *% H* | *% N* |
| *calculé* | *65,08* | *3,07* | *4,74* |
| *trouvé* | *59,30* | *3,77* | *4,39* |

**EXEMPLE 5 : 3-(3,4,5-Triméthoxyphényl)-8*H*-thiéno-[2,3-*b*]pyrrolizine-8-one**

On procède comme dans l'exemple 1, des stades A à F, en utilisant comme substrat dans le stade A, le 2-(3,4,5-triméthoxyphényl)-acétonitrile
*Point de fusion : 180°C*

| *Microanalyse élémentaire :* | | | |
|---|---|---|---|
| | *% C* | *% H* | *% N* |
| *calculé* | *63,33* | *4,43* | *4,10* |
| *trouvé* | *63,26* | *4,86* | *4,22* |

**EXEMPLE 6 : 3-(2-Hydroxyphényl)-8*H*-thiéno-[2,3-*b*]pyrrolizine-8-one**

Une solution, de 1,1 mmol du composé de l'exemple 1 et de 1,1 mmol de tribromure de bore dans le chloroforme, est agitée 30 minutes à température ambiante. Après addition de 100 ml d'eau, le précipité formé est filtré, séché puis recristallisé dans l'éthanol permettant d'isoler le produit attendu.
*Point de fusion : 192°C*

| *Microanalyse élémentaire :* | | | |
|---|---|---|---|
| | *% C* | *% H* | *% N* |
| *calculé* | *67,40* | *3,39* | *5,24* |
| *trouvé* | *66,49* | *3,25* | *5,48* |

**EXEMPLE 7 : 3-(3-Hydroxyphényl)-8*H*-thiéno-[2,3-*b*]pyrrolizine-8-one**

On procède comme dans l'exemple 6 en utilisant comme substrat le produit obtenu dans l'exemple 2.
*Point de fusion : > 260°C*

| *Microanalyse élémentaire :* | | | |
|---|---|---|---|
| | *% C* | *% H* | *% N* |
| *calculé* | *67,40* | *3,39* | *5,24* |
| *trouvé* | *64,19* | *3,24* | *4,86* |

**EXEMPLE 8 : 3-(3,4-Dihydroxyphényl)-8*H*-thiéno-[2,3-*b*]pyrrolizine-8-one**

On procède comme dans l'exemple 6 en utilisant comme substrat le produit obtenu dans l'exemple 3.
*Point de fusion : > 260°C*

**EXEMPLE 9 : 3-(4-Ethoxyphényl)-8*H*-thiéno-[2,3-*b*]pyrrolizine-8-one**

***Stade G : 3-(4-Métoxyphényl)-8H-thiéno-[2,3-b]pyrrolizine-8-one***

Le produit est obtenu selon les conditions opératoires décrites dans la demande de brevet EP718299.

***Stade H : 3-(4-Ethoxyphényl)-8H-thiéno-[2,3-b]pyrrolizine-8-one***

A une solution de 1,4 mmol de sodium dans 100 ml de méthanol sont additionnées 1,4 mmol du composé obtenu dans le stade G et 15 ml de bromure d'éthyle. Le mélange réactionnel est ensuite chauffé jusqu'à disparition complète du substrat en chromatographie sur couche mince (dichlorométhane/méthanol : 9/1). Le solvant est ensuite évaporé sous pression réduite, puis le solide obtenu est agité dans l'hydroxyde de sodium aqueuse 0,5 N Après filtration et lavage à l'eau, les cristaux sont séchés puis recristallisés dans un mélange méthanol/éthanol : 1/2, permettant d'isoler le produit attendu.
*Point de fusion : 200°C*

| *Microanalyse élémentaire :* | | | |
|---|---|---|---|
| | *% C* | *% H* | *% N* |
| *calculé* | *69,13* | *4,44* | *4,74* |
| *trouvé* | *69,07* | *4,44* | *4,96* |

**EXEMPLE 10 : 3-(4-Propoxyphényl)-8*H*-thiéno-[2,3-*b*]-pyrrolizine-8-one**

On procède comme dans le stade H de l'exemple 9 en utilisant comme réactif le bromure de *n*-propyle. Le produit est recristallisé dans un mélange méthanol/isopropanol : 1/2.
*Point de fusion : 172°C*

| *Microanalyse élémentaire :* | | | |
|---|---|---|---|
| | *% C* | *% H* | *% N* |
| *calculé* | *69,88* | *4,89* | *4,53* |
| *trouvé* | *69,06* | *4,65* | *4,87* |

**EXEMPLE 11 : 3-(4-Butoxyphényl)-8*H*-thiéno-[2,3-*b*]pyrrolizine-8-one**

On procède comme dans le stade H de l'exemple 9, en utilisant comme réactif le bromure de *n*-butyle. Le produit est recristallisé dans l'éthanol.
*Point de fusion : 129°C*

| *Microanalyse élémentaire :* | | | |
|---|---|---|---|
| | *% C* | *% H* | *% N* |
| *calculé* | *70,56* | *5,30* | *4,33* |
| *trouvé* | *69,84* | *5,28* | *4,65* |

**EXEMPLE 12 : 6-Bromo-3-(4-méthoxyphényl)-8*H*-thiéno-[2,3-*b*]pyrrolizine-8-one**

A une solution de 1,8 mmol de 3-(4-méthoxyphényl)-8*H*-thiéno-[2,3-*b*]pyrrolizine-8-one dans 50 ml de chloroforme sont additionnés 2,1 mmol de brome. Après deux heures d'agitation, le solvant est évaporé sous pression réduite. Une chromatographie sur gel de silice (dichlorométhane) permet d'isoler le produit attendu.
*Point de fusion : 186°C*

| *Microanalyse élémentaire :* | | | |
|---|---|---|---|
| | %C | *%H* | *%N* |
| *calculé* | *53,35* | *2,80* | *3,89* |
| *trouvé* | *52,58* | *2,82* | *4,12* |

**EXEMPLE 13 : 2,6-Dibromo-2(4-méthoxyphényl)8*H*-thiéno-[2,3-*b*]pyrrolizine-8-one**

Le produit est isolé lors de la chromatographie sur gel de silice effectuée sur le composé de l'exemple 12.
*Point de fusion* : *202°C*

| *Microanalyse élémentaire :* | | | |
|---|---|---|---|
| | *% C* | *%H* | *%N* |
| *calculé* | *43,76* | *2,07* | *3,19* |
| *trouvé* | *43,48* | *2,02* | *3,58* |

**EXEMPLE 14 : 3-(3-Hydroxy-4-méthoxyphényl)8*H*-thiéno-[2,3-*b*]pyrrolizine-8-one**

Une solution de 0,6 mmol du composé de l'exemple 3 et d'un excès de chlorure d'aluminium dans le chloroforme est agitée à 50°C pendant 30 minutes. Après concentration sous pression réduite, le résidu est agité dans l'eau pendant 30 minutes pour fournir des cristaux rouges. Après filtration, une chromatographie sur gel de silice (chloroforme) permet d'isoler le produit attendu.
*Point de fusion : 180°C*

| *Microanalyse élémentaire :* | | | |
|---|---|---|---|
| | %C | %H | %N |
| *calculé* | *64,63* | *3,73* | *4,71* |
| *trouvé* | *62,89* | *3,84* | *4,69* |

**EXEMPLE 15 : 6-Nitro-3-(4-méthoxyphényl)-8*H*-thiéno-[2,3-*b*]pyrrolizine-8-one**

Une solution de 1 mmol du composé obtenu dans l'exemple 12 dans 50 ml de nitrométhane est agitée 12 heures à température ambiante, puis le mélange réactionnel est jeté sur une solution de potasse 1M. Après extraction au dichlorométhane, les phases organiques sont séchées sur sulfate de sodium, filtrées et concentrées sous pression réduite. Une chromatographie sur gel de silice (chloroforme) permet d'isoler le produit attendu.

**EXEMPLE 16 : 6-Amino-3-(4-méthoxyméthyl)-8*H*-thiéno-[2,3-*b*]pyrrolizin-8-one**

Une solution contenant 1 équivalent du composé obtenu dans l'exemple 14 dans 30 ml de méthanol et 100 mg de Palladium sur charbon à 10 % est chauffée pendant 2 heures à 40°C. Après retour à température ambiante, le milieu réactionnel est filtré sur célite, puis concentré sous pression réduite, permettant d'isoler le produit attendu.

**EXEMPLE 17 : 6-Méthoxy-3-(4-méthoxyphényl)-8*H*-thiéno-[2,3-*b*|pyrrolizine-8-one**

1 équivalent du composé obtenu dans l'exemple 12 est agité, à température ambiante, dans une solution de méthylate de sodium. Après 12 heures de réaction, le milieu réactionnel est concentré sous pression réduite. Le résidu est repris au dichlorométhane, puis la phase organique est lavée par une solution saturée au NaCl, séchée sur sulfate de sodium puis concentrée sous pression réduite. Une chromatographie sur gel de silice (dichlorométhane) permet d'isoler le produit attendu.

**EXEMPLE 18 : Butyrate de 2-(butyryloxy)-4-(8-oxo-8*H*-thiéno[2,3-*b*]pyrrolizin-3-yl)phényl**

A une solution de 1,7 mmoles du composé de l'exemple 8 dans 50 ml de tétrahydrofurane sont ajoutés goutte à goutte 2,2 équivalents d'anhydride butanoïque. Après une heure de reflux puis refroidissement, quelques gouttes

d'eau et 50 ml de soude à 5 % sont additionnés au milieu réactionnel. La phase organique est ensuite extraite, séchée, filtrée puis évaporée sous pression réduite permettant d'isoler le produit attendu.

| *Microanalyse élémentaire :* | | | |
|---|---|---|---|
| | *% C* | *% H* | *% N* |
| *calculé* | *65,23* | *4,99* | *3,30* |
| *trouvé* | *65,11* | *4,93* | *4,02* |

**<u>EXEMPLE 19</u> : Pivalate de 2-hydroxy-4-(8-oxo-8*H*-thiéno(2,3-*b*]pyrrolizin-3-yl) phényl**

On procède comme dans l'exemple 18 en utilisant comme réactif l'anhydride 1,1-diméthylpropanoïque.

| *Microanalyse élémentaire :* | | | |
|---|---|---|---|
| | *% C* | *%H* | *%N* |
| *calculé* | *65,38* | *4,66* | *3,81* |
| *trouvé* | *65,18* | *4,69* | *3,73* |

**<u>EXEMPLE 20</u> : Acétate de 2-(acétyloxy)-4-(8-oxo-8*H*-thiéno[2,3-*b*]pyrrolizin-3-yl) phényl**

On procède comme dans l'exemple 18 en utilisant comme réactif l'anhydride acétique

| *Microanalyze élémentaire :* | | | |
|---|---|---|---|
| | *%C* | *% H* | *%N* |
| *calculé* | *62,11* | *3,56* | *3,81* |
| *trouvé* | *62,18* | *3,53* | *3,97* |

**<u>EXEMPLE 21</u> : Propionate de 4-(8-oxo-8*H*-thiéno[2,3-*b*]pyrrolizin-3-yl)-2-(propionyloxy)phényl**

On procède comme dans l'exemple 18 en utilisant comme réactif l'anhydride propanoïque.

| *Microanalyse élémentaire :* | | | |
|---|---|---|---|
| | *%C* | *%H* | *%N* |
| *calculé* | *63,78* | *4,33* | *3,54* |
| *trouvé* | *63,50* | *4,29* | *3,51* |

**<u>EXEMPLE 22</u> : 3-[3-(benzyloxy)-4-méthoxyphényl]-8*H*-thiéno[2,3-*b*]pyrrolizin-8-one**

***<u>Préparation A</u> : 2-[3-(Benzyloxy)-4métoxyphényl]acétonitrile***

***<u>Stade 1</u> : 3-Benzyloxy-4-méthoxybenzaldéhyde***

A une suspension de 0,33 mol de 3-hydroxy-4-méthoxybenzaldéhyde dans 400 ml de méthanol sont additionnés 1,2 équivalents de $K_2CO_3$ et 1,2 équivalents de bromure de benzyle. Après 24 heures de reflux, le milieu réactionnel est filtré à chaud puis la phase organique est évaporée sous pression réduite. Le résidu isolé est repris dans 300 ml de chloroforme puis lavé à l'eau. Après extraction, séchage et filtration, une évaporation sous pression réduite de la phase organique permet d'isoler le produit attendu.

***<u>Stade 2</u> : [3-(Benzyloxy)-4-méthoxyphényl]méthanol***

Une solution de 0,35 mol du composé obtenu dans le stade 1 dans 400 ml de méthanol et 1,5 équivalents de borohydrure de sodium est agitée 6 heures à température ambiante, puis 500 ml d'eau acidulée sont lentement ajoutées. Le précipité obtenu est repris dans l'éther, puis la phase organique est extraite, séchée, filtrée puis évaporée

sous pression réduite permettant d'isoler le produit attendu.

***<u>Stade 3</u> : 2-Benzyloxy-4-chlorométhyl-1-méthoxybenzène***

Une solution de 0,32 mol du composé obtenu dans le stade 2 et 1,2 équivalents de chlorure de thionyle dans 300 ml de dioxane est agitée à température ambiante. Après 1 heure de réaction, 500 ml d'eau sont additionnés. Le précipité formé est filtré, rincé à l'eau, puis séché permettant d'isoler le produit attendu.

***<u>Stade 4</u> : 2-[3-(Benzyloxy)-4-méthoxyphényl]acétonitrile***

Une solution de 0,22 mol du composé obtenu au stade 3 dans 300 ml d'acétonitrile et 1,1 équivalents de cyanure de tétraéthylammonium est agitée une journée à température ambiante, puis 500 ml d'une solution d'hydrogénocarbonate de sodium à 5 % sont additionnés. Le précipité obtenu est repris dans l'éther, puis la phase organique est extraite, séchée, filtrée et évaporée sous pression réduite, permettant d'isoler le produit attendu.

| *Microanalyse élémentaire :* | | | |
|---|---|---|---|
| | *% C* | *% H* | *% N* |
| *calculé* | *75,86* | *5,96* | *5,52* |
| *trouvé* | *75,83* | *6,12* | *5,55* |

Le produit de la Préparation A est alors soumis aux réactions décrites dans l'exemple 1, des stades A à F, permettant d'isoler le produit "titre" attendu.

| *Microanalyse élémentaire :* | | | |
|---|---|---|---|
| | *% C* | *% H* | *% N* |
| *calculé* | *71,30* | *4,42* | *3,62* |
| *trouvé* | *71,52* | *4,67* | *3,77* |

**<u>EXEMPLE 23</u> : 3-[4(Benzyloxy)-3-méthoxyphényl]-8*H*-thiéno[2,3-*b*]pyrrolizin-8-one**

***<u>Préparation B</u> : 2-[4-(Benzyloxy)-3-méthoxyphényl]acétonitrile***

On procède comme dans la Préparation A, des stades 1 à 4, en utilisant comme substrat au stade 1 le 4-hydroxy-3-méthoxybenzaldéhyde.

| *Microanalyse élémentaire :* | | | |
|---|---|---|---|
| | *% C* | *% H* | *% N* |
| *calculé* | *75,86* | *5,96* | *5,52* |
| *trouvé* | *75,62* | *6,02* | *5,88* |

Le produit de la Préparation B est alors soumis aux réactions décrites dans l'exemple 1, des stades A à F, permettant d'isoler le produit "titre" attendu.

| *Microanalyse élémentaire :* | | | |
|---|---|---|---|
| | *% C* | *% H* | *% N* |
| *calculé* | *71,30* | *4,42* | *3,62* |
| *trouvé* | *70,87* | *4,32* | *3,64* |

**<u>EXEMPLE 24</u> : 3-(4-Hydroxy-3-méthoxyphényl)-8*H*-thiéno(2,3-*b*)pyrrolizin-8-one**

1 mmol du composé de l'exemple 23 dissous dans un excès d'acide bromhydrique dans de l'acide acétique à 33 % est agité 30 minutes à température ambiante. Après addition de 100 ml d'eau, un précipité est formé, celui-ci

étant filtré puis séché. Une chromatographie sur gel de silice (Acétate d'éthyel/hexane : 1/2) permet d'isoler le produit attendu.

**EXEMPLE 25 : Acétate de 2-méthoxy-5-(8-oxo-8*H*-thiéno[2,3-*b*]pyrrolizin-3-yl) phényl**

On procède comme dans l'exemple 18 en utilisant comme substrat le composé de l'exemple 24 et en réalisant la réaction dans l'acide acétique en présence d'anhydride acétique.

| *Microanalyse élémentaire :* | | | |
|---|---|---|---|
| | *% C* | *% H* | *% N* |
| *calculé* | *63,70* | *3,86* | *4,12* |
| *trouvé* | *63,25* | *4,46* | *4,37* |

**ETUDE PHARMACOLOGIQUE DES COMPOSES DE L'INVENTION**

**EXEMPLE 26 : Cytotoxicité in vitro**

Six lignées cellulaires ont été utilisées :

- 2 leucémies murines, P388 et L1210,
- 1 carcinome pulmonaire humain, non à petites cellules, A549,
- 1 carcinome épidermoïde humain, KB-3-1 et la lignée résistante correspondante, KB-A1 dont la résistance multidrogue a été induite par l'adriamycine (ADR),
- 1 carcinome ovarien humain, IGROV1.

Les cellules sont cultivées dans du milieu RPMI 1640 complet contenant 10 % de sérum de veau foetal, 2 mM de glutamine, 50 unités/ml de pénicilline, 50 µg/ml de streptomycine et 10 mM d'Hepes, pH = 7,4. Les cellules sont réparties dans des microplaques et exposées aux composés cytotoxiques. Les cellules sont ensuite incubées pendant 2 jours (P388, L1210) ou 4 jours (A549, KB-A1, KB-3-1, IGROV1). Le nombre de cellules viables est ensuite quantifié par un essai colorimétrique, le Microculture Tetrazolium Assay (*Cancer Res.* **1987**, 47, 939-942).

Les résultats sont exprimés en $IC_{50}$, concentration en cytotoxique qui inhibe à 50 % la prolifération des cellules traitées. Lors de ces tests, le composé de l'exemple 8 présente les $IC_{50}$ mentionnées dans le tableau ci-dessous :

| | $IC_{50}$ nM | | | | | |
|---|---|---|---|---|---|---|
| Composés testés | P388 | L1210 | A549 | KB-3-1 | KB-A1 | IGROV1 |
| Exemple 8 | 356 | 222 | 122 | 33 | 22 | 56 |

Le composé de l'exemple 8 est plus puissant sur les cellules issues de tumeurs solides humaines, que sur les deux leucémies murines, résultat particulièrement surprenant et intéressant.

**EXEMPLE 27 : Action sur le cycle cellulaire**

Les cellules L1210 sont incubées pendant 21 heures à 37°C en présence de différentes concentrations en produits testés. Les cellules sont ensuite fixées par de l'éthanol à 70 % (V/V), lavées 2 fois dans du PBS et incubées 30 minutes à 20°C dans du PBS contenant 100 µg/ml de RNAse et 50 µg/ml de iodure de propidium. Les résultats sont exprimés en pourcentage des cellules accumulées en phase G2+M après 21 heures par rapport au témoin (témoin : 20 %). Le composé de l'exemple 8 induit une accumulation à 80-90 % des cellules en phase G2+M après 21 heures à une concentration de 500 nM.

**EXEMPLE 28 : Activité *in vivo :* activité antitumorale des composés sur la leucémie P388**

La lignée P388 (leucémie murine) a été fournie par le National Cancer Institue (Frederick USA). Les cellules tumorales ($10^6$ cellules) ont été inoculées au jour 0 dans la cavité péritonéale de souris BDF1 femelles (Iffa-Credo, France) pesant de 18 à 20 g (groupes de 6 animaux). Les produits ont été administrés une fois par jour pendant 4 jours (J1-4) aux doses indiquées, par voie intrapéritonéale.

L'activité antitumorale est exprimée en % de T/C :

$$\% \text{ T/C} = \frac{\text{Temps de survie médian des animaux traités}}{\text{Temps de survie médian des animaux contrôlés}} \times 100$$

Le composé de l'exemple 8 est actif à partir de la dose de 25 mg/kg et il peut permettre d'augmenter la survie des animaux traités de 70 %.

**EXEMPLE 29 : Activité in vivo : activité antitumorale des composés sur le carcinome ovarien IGROV1**

Les cellules tumorales IGROVI ($10^7$ cellules) ont été incubées au jour 0 dans la cavité péritonéale de souris nude BABL/C (groupes de 5 animaux). Les produits ont été administrés une fois par semaine aux jours 4, 11 et 18, par voie intrapéritonéale. L'activité antitumorale est exprimée en % de T/C comme défini précédemment. Le composé de l'exemple 8 est actif à la dose de 200 mg/kg et il augmente la survie des animaux traités de 60 % (T/C = 160 %).

**EXEMPLE 30 : Composition pharmaceutique : comprimés**

| Formule de préparation pour 1000 comprimés dosés à 10 mg | |
|---|---|
| Composé de l'exemple 8 | 10 g |
| Hydroxypropylcellulose | 2 g |
| Amidon de blé | 10 g |
| Lactose | 100 g |
| Stéarate de magnésium | 2 g |
| Talc | 2 g |

## Revendications

**1.** Composés de formule (I) :

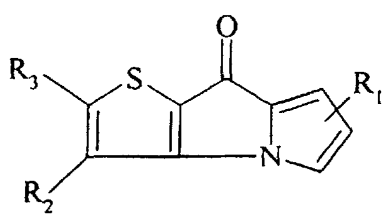

(I)

dans laquelle :

$R_1$ représente un atome d'hydrogène, d'halogène, un groupement alkyle ($C_1$-$C_6$) linéaire ou ramifié, nitro, hydroxy, alkoxy ($C_1$-$C_6$) linéaire ou ramifié, trihalogénoalkyle ($C_1$-$C_6$) linéaire ou ramifié, trihalogénoalkoxy ($C_1$-$C_6$) linéaire ou ramifié, ou un groupement amino (éventuellement substitué par un ou deux groupements, identiques ou différents, indépendamment l'un de l'autre, alkyle ($C_1$-$C_6$) linéaire ou ramifié),

$R_2$ représente un groupement aryle éventuellement substitué ou hétéroaryle éventuellement substitué,

$R_3$ représente un atome d'hydrogène, d'halogène, un groupement alkyle ($C_1$-$C_6$) linéaire ou ramifié, nitro, hy-

droxy, alkoxy ($C_1$-$C_6$) linéaire ou ramifié, trihalogénoalkyle ($C_1$-$C_6$) linéaire ou ramifié, trihalogénoalkoxy ($C_1$-$C_6$) linéaire ou ramifié, ou un groupement amino (éventuellement substitué par un ou deux groupements, identiques ou différents, indépendamment l'un de l'autre, alkyle ($C_1$-$C_6$) linéaire ou ramifié),

leurs isomères ainsi que leurs sels d'addition à un acide ou à une base pharmaceutiquement acceptable, étant entendu que lorsque $R_1$ et $R_3$ représentent simultanément un atome d'hydrogène, alors $R_2$ ne peut pas représenter un groupement phényle éventuellement substitué en position *para* par un atome de brome, de chlore, de fluor, un groupement méthoxy ou hydroxy.

**2.** Composés de formule (I) selon la revendication 1, **caractérisés en ce que** $R_2$ représente un groupement phényle substitué par au moins un groupement, choisi de façon identique ou différente, parmi halogène, alkyle ($C_1$-$C_6$) linéaire ou ramifié, nitro,. cyano, hydroxy, alkoxy ($C_1$-$C_6$) linéaire ou ramifié, trihalogénoalkoxy ($C_1$-$C_6$) linéaire ou ramifié, amino, mono ou dialkylamino ($C_1$-$C_6$) linéaire ou ramifié, acyle ($C_1$-$C_6$) linéaire ou ramifié, carboxy, alkoxycarbonyle ($C_1$-$C_6$) linéaire ou ramifié, méthylènedioxy, éthylènedioxy, alkylcarbonyloxy ($C_1$-$C_6$) linéaire ou ramifié, et arylalkoxy ($C_1$-$C_6$) linéaire ou ramifié, leurs isomères ainsi que leurs sels d'addition à un acide ou à une base pharmaceutiquement acceptable.

**3.** Composés de formule (I) selon l'une quelconque des revendications 1 ou 2, **caractérisés en ce que** $R_2$ représente un groupement phényle substitué par au moins un groupement choisi parmi hydroxy, alkoxy ($C_1$-$C_6$) linéaire ou ramifié, et alkylcarbonyloxy ($C_1$-$C_6$) linéaire ou ramifié, leurs isomères ainsi que leurs sels d'addition à un acide ou à une base pharmaceutiquement acceptable.

**4.** Composés de formule (I) selon la revendication 1 **caractérisés en ce que** $R_1$ représente un atome d'hydrogène et $R_3$ représente un atome d'hydrogène, leurs isomères ainsi que leurs sels d'addition à un acide ou à une base pharmaceutiquement acceptable.

**5.** Composé de formule (I) selon la revendication 1 qui est le 3-(3,4-dihydroxyphényl)-8*H*-thiéno-[2,3-*b*]pyrrolizine-8-one.

**6.** Composé de formule (I) selon la revendication 1 qui est l'acétate de 2-méthoxy-5-(8-oxo-8*H*-thiéno[2,3-*b*]pyrrolizin-3-yl)phényl.

**7.** Procédé de préparation des composés de formule (I), **caractérisé en ce qu'**on utilise comme produit de départ un composé de formule (II) :

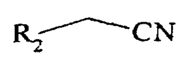

(II)

dans laquelle $R_2$ est tel que défini dans la formule (I),
composés de formule (II) que l'on fait réagir en présence d'un métal alcalin, avec du formiate d'éthyle, pour conduire aux composés de formule (III) :

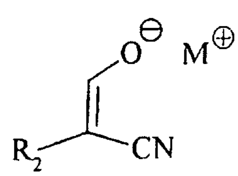

(III)

dans laquelle $R_2$ est tel que défini précédemment et $M^+$ représente un cation alcalin, tel que le cation sodium ou potassium,
composés de formule (III) que l'on place dans des conditions d'addition nucléophile en présence de chlorure de phénylsulfonyle, pour conduire aux composés de formule (IV) :

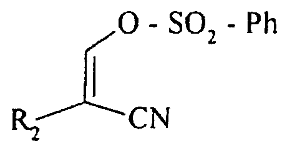

(IV)

dans laquelle $R_2$ est tel que défini précédemment, composés de formule (IV) sur lequel on fait réagir du thioglycolate de méthyle, pour conduire aux composés de formule (V) :

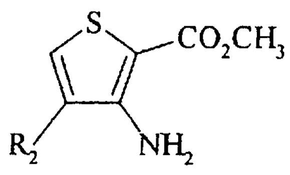

(V)

dans laquelle $R_2$ est tel que défini précédemment, composés de formule (V) dont on protège le groupement amino, selon des méthodes classiques de synthèse organique, puis que l'on fait réagir avec un composé de formule (VI) :

$$R_3 - X \quad (VI)$$

dans laquelle X représente un groupement libérable et $R_3$ est tel que défini dans la formule (I), puis dont on déprotège la fonction amino, pour conduire aux composés de formule (VII) :

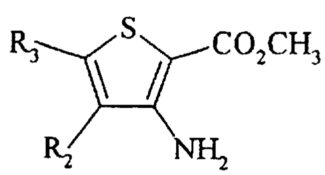

(VII)

dans laquelle $R_2$ et $R_3$ sont tels que définis dans la formule (I), composés de formule (VII) que l'on place en présence de diméthoxytétrahydrofurane et de chlorure de 4-chloropyridinium, pour conduire aux composés de formule (VIII) :

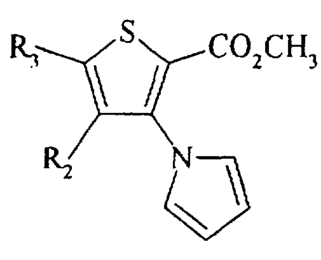

(VIII)

dans laquelle $R_2$ et $R_3$ sont tels que définis précédemment, puis que l'on fait réagir en présence de pyrrolidine, pour conduire aux composés de formule (IX) :

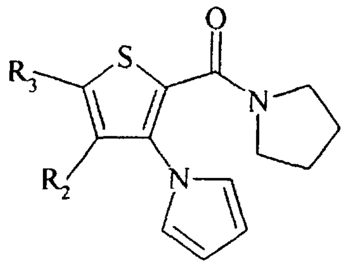

(IX)

dans laquelle $R_2$ et $R_3$ sont tels que définis précédemment,
composés de formule (IX) que l'on cyclise avec de l'oxychlorure de phosphore, pour conduire aux composés de formule (I/a), cas particulier des composés de formule (I) :

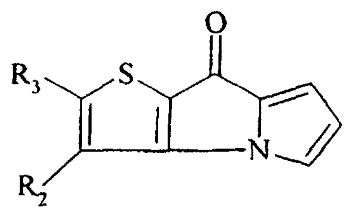

(I/a)

dans laquelle $R_2$ et $R_3$ sont tels que définis précédemment,
composés de formule (I/a) dont on fonctionnalise le noyau pyrrole selon des conditions classiques de synthèse organique, pour conduire aux composés de formule (I/b), cas particulier des composés de formule (I) :

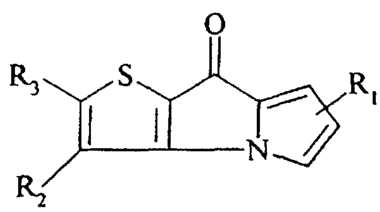

(I/b)

dans laquelle $R_2$, $R_3$ et $R_1$ sont tels que définis dans la formule (I), excepté que $R_1$ ne peut pas représenter un atome d'hydrogène,
les composés (I/a) et (I/b) forment l'ensemble des composés de l'invention, que l'on purifie, le cas échéant, selon une technique classique de purification, qui peuvent, si on le désire, ètre séparés en leurs différents isomères selon une technique classique de séparation, et que l'on transforme, le cas échéant, en leurs sels d'addition à un acide ou à une base pharmaceutiquement acceptable.

**8.** Compositions pharmaceutiques contenant comme principe actif au moins un composé de formule (I) selon l'une quelconque des revendications 1 à 6, seul ou en combinaison avec un ou plusieurs excipients ou véhicules inertes, non toxiques, pharmaceutiquement acceptables.

**9.** Compositions pharmaceutiques selon la revendication 8 contenant au moins un principe actif selon l'une quelconque des revendications 1 à 6, utiles dans le traitement du cancer.

**Patentansprüche**

**1.** Verbindungen der Formel (I):

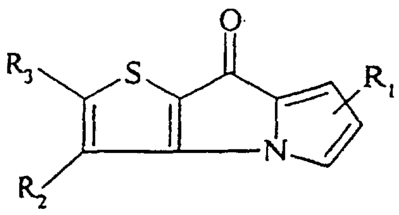

(I)

in der:

$R_1$ ein Wässerstoffatom, Halogenatom, eine geradkettige oder verzweigte ($C_1$-$C_6$)-Alkylgruppe, Nitrogruppe, Hydroxygruppe, geradkettige oder verzweigte ($C_1$-$C_6$)-Alkoxygruppe, geradkettige oder verzweigte ($C_1$-$C_6$) -Trihalogenalkylgruppe, geradkettige oder verzweigte ($C_1$-$C_6$)-Trihalogenalkoxygruppe oder eine Aminogruppe (die gegebenenfalls durch eine oder zwei geradkettige oder verzweigte ($C_1$-$C_6$)-Alkylgruppen unabhängig voneinander substituiert ist),

$R_2$ eine gegebenenfalls substituierte Arylgruppe oder gegebenenfalls substituierte Heteroarylgruppe und

$R_3$ ein Wasserstoffatom, Halogenatom, eine geradkettige oder verzweigte ($C_1$-$C_6$)-Alkylgruppe, Nitrogruppe, Hydroxygruppe, geradkettige oder verzweigte ($C_1$-$C_6$)-Alkoxygruppe, geradkettige oder verzweigte ($C_1$-$C_6$) -Trihalogenalkylgruppe, geradkettige oder verzweigte ($C_1$-$C_6$)-Trihalogenalkoxygruppe oder eine Aminogruppe (die gegebenenfalls durch eine oder zwei gleichartige oder verschiedene geradkettige oder verzweigte ($C_1$-$C_6$)-Alkylgruppen unabhängig voneinander substituiert ist),

bedeuten,
deren Isomere sowie deren Additionssalze mit einer pharmazeutisch annehmbaren Säure oder Base, mit der Maßgabe, daß, wenn $R_1$ und $R_3$ gleichzeitig Wasserstoffatome bedeuten, $R_2$ keine Phenylgruppe darstellt, die gegebenenfalls in der *para*-Stellung durch ein Bromatom, Chloratom, Fluoratom, eine Methoxygruppe oder eine Hydroxygruppe substituiert ist.

2. Verbindungen der Forme (I) nach Anspruch 1, **dadurch gekennzeichnet, daß** $R_3$ eine Phenylgruppe bedeutet, die durch mindestens eine Gruppe substituiert ist, die gleichartig oder verschieden sind und ausgewählt sind aus Halogen, geradkettigem oder verzweigtem ($C_1$-$C_6$)-Alkyl, Nitro, Cyano, Hydroxy, geradkettige oder verzweigtem ($C_1$-$C_6$)-Alkoxy, geradkettigem oder verzweigtem ($C_1$-$C_6$)-Trihalogenalkoxy, Amino, geradkettigem oder verzweigtem Mono- oder Di-($C_1$-$C_6$)-alkyl-amino, geradkettigem oder verzweigtem ($C_1$-$C_6$)-Acyl, Carboxy, geradkettigem oder verzweigtem ($C_1$-$C_6$)-Alkoxycarbonyl, Methylendioxy, Ethylendioxy, geradkettigem oder verzweigtem ($C_1$-$C_6$) -Alkylcarbonyloxy und geradkettigem oder verzweigtem ($C_1$-$C_6$)-Arylalkoxy, deren Isomere sowie deren Additionssalze mit einer pharmazeutisch annehmbaren Säure oder Base.

3. Verbindungen der Formel (I) nach einem der Ansprüche 1 oder 2, **dadurch gekennzeichnet, daß** $R_2$ eine Phenylgruppe bedeutet, die durch mindestens eine Gruppe ausgewählt aus Hydroxy, geradkettigem oder verzweigtem ($C_1$-$C_6$)-Alkoxy und geradkettigem oder verzweigtem ($C_1$-$C_6$)-Alkylcarbonyloxy substituiert ist, deren Isomere sowie deren Additionssalze mit einer pharmazeutisch annehmbaren Säure oder Base.

4. Verbindungen der Formel (I) nach Anspruch 1, **dadurch gekennzeichnet, daß** $R_1$ ein Wasserstoffatom und $R_3$ ein Wasserstoffatom bedeuten, deren Isomere sowie deren Additionssalze mit einer pharmazeutisch annehmbaren Säure oder Base.

5. Verbindung der Formel (I) nach Anspruch 1, nämlich 3-(3,4-Dihydroxyphenyl)-8*H*-thieno(2,3-*b*]pyrrolizin-8-on.

6. Verbindung der Formel (I) nach Anspruch 1, nämlich 2-Methoxy-5-(8-oxo-8*H*-thieno(2,3-*b*]pyrrolizin-3-yl)-phenylacetat.

7. Verfahren zur Herstellung der Verbindungen der Formel (I), **dadurch gekennzeichnet, daß** man als Ausgangsprodukt eine Verbindung der Formel (II) verwendet:

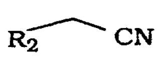

(II)

in der $R_2$ die bezüglich der Formel (I) angegebenen Bedeutungen besitzt,
welche Verbindung der Formel (II) man in Gegenwart eines Alkalimetalls mit Ameisensäureethylester umsetzt zur Bildung der Verbindungen der Formel (III):

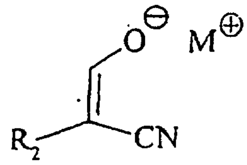

(III)

in der $R_2$ die oben angegebenen Bedeutungen besitzt und $M^-$ ein Alkalikation, wie das Natrium- oder Kaliumkation darstellt,
welche Verbindungen der Formel (III) man in Gegenwart von Phenylsulfonylchlorid den Bedingungen der nucleophilen Addition aussetzt zur Bildung der Verbindungen der Formel (IV):

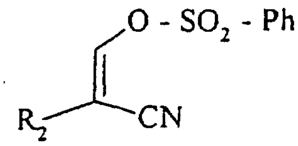

(IV)

in der $R_2$ die oben angegebenen Bedeutungen besitzt,
welche Verbindungen der Formel (IV) man mit Thioglykolsäuremethylester umsetzt zur Bildung der Verbindungen der Formel (V):

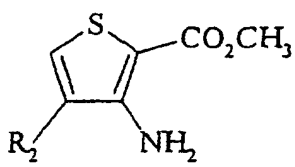

(V)

in der $R_2$ die oben angegebenen Bedeutungen besitzt,
bei welchen Verbindungen der Formel (V) man die Aminogruppe mit Hilfe klassischer Methoden der organischen Synthese schützt und dann mit einer Verbindung der Formel (VI) umsetzt:

$$R_3 - X \qquad (VI)$$

in der X eine freisetzbare Gruppe darstellt und $R_3$ die bezüglich der Formel (I) angegebenen Bedeutungen besitzt, und man dann die Aminogruppe von ihrer Schutzgruppe befreit,
zur Bildung der Verbindungen der Formel (VII):

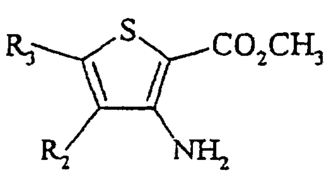

(VII)

in der $R_2$ und $R_3$ die bezüglich der Formel (I) angegebenen Bedeutungen besitzen, welche Verbindungen der Formel (VII) man mit Dimethoxytetrahydrofuran und 4-Chlorpyridiniumchlorid umsetzt zur Bildung der Verbindun-

gen der Formel (VIII):

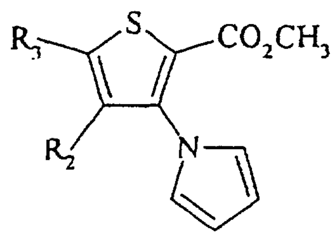

(VIII)

in der $R_2$ und $R_3$ die oben angegebenen Bedeutungen besitzen,
welche man in Gegenwart von Pyrrolidin umsetzt zur Bildung der Verbindungen der Formel (IX):

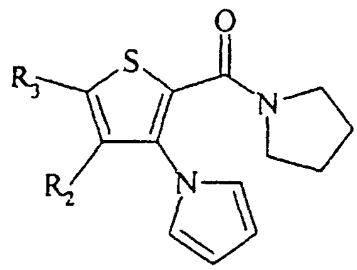

(IX)

in der $R_2$ und $R_3$ die oben angegebenen Bedeutungen besitzen,
welche Verbindungen der Formel (IX) man mit Phosphoroxidchlorid cyclisiert zur Bildung der Verbindungen der Formel (I/a), einem Sonderfall der Verbindungen der Formel (I):

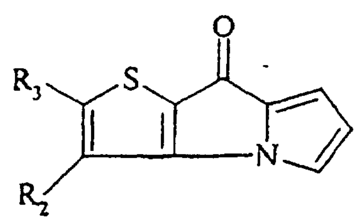

(I/a)

in der $R_2$ und $R_3$ die oben angegebenen Bedeutungen besitzen,
welche Verbindungen der Formel (I/a) man am Pyrrolkern unter Anwendung klassischer Bedingungen der organischen Synthese funktionalisiert zur Bildung der Verbindungen der Formel (I/b), einem Sonderfall der Verbindungen der Formel (I):

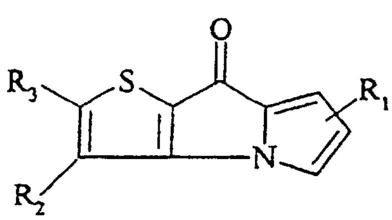

(I/b)

in der $R_2$, $R_3$ und $R_1$ die bezüglich der Formel (I) angegebenen Bedeutungen besitzen, mit der Maßgabe, daß $R_1$ kein Wasserstoffatom bedeuten kann,
welche Verbindungen (I/a) und (I/b) die Gesamtheit der erfindungsgemäßen Verbindungen bilden, welche man gegebenenfalls mit Hilfe einer klassischen Reinigungsmethode reinigt, welche gewünschtenfalls mit Hilfe einer klassischen Trennmethode in ihre verschiedenen Isomeren aufgetrennt werden können und welche man gewünschtenfalls in ihre Additionssalze mit einer pharmazeutisch annehmbaren Säure oder Base umwandelt.

**8.** Pharmazeutische Zubereitungen enthaltend als Wirkstoff mindestens eine Verbindung der Formel (I) nach irgendeinemd er Ansprüche 1 bis 6 allein oder in Kombination mit einem oder mehreren inerten, nichttoxischen, pharmazeutisch annehmbaren Trägermaterialien oder Bindemitteln.

**9.** Pharmazeutische Zubereitungen nach Anspruch 8, enthaltend mindestens einen Wirkstoff nach einem der Ansprüche 1 bis 6 zur Behandlung von Krebs.

**Claims**

**1.** Compounds of formula (I) :

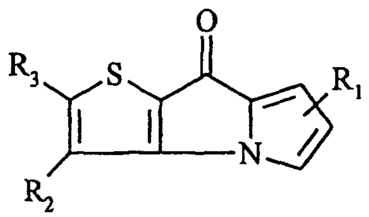

(I),

wherein :

$R_1$ represents a hydrogen atom, a halogen atom, a linear or branched ($C_1$-$C_6$)alkyl group, a nitro group, a hydroxy group, a linear or branched ($C_1$-$C_6$)alkoxy group, a linear or branched ($C_1$-$C_6$)trihaloalkyl group, a linear or branched ($C_1$-$C_6$)trihaloalkoxy group, or an amino group (optionally substituted by one or two linear or branched ($C_1$-$C_6$)-alkyl groups, which may be identical or different, each independently of the other),

$R_2$ represents an optionally substituted aryl group or an optionally substituted heteroaryl group,

$R_3$ represents a hydrogen atom, a halogen atom, a linear or branched ($C_1$-$C_6$)alkyl group, a nitro group, a hydroxy group, a linear or branched ($C_1$-$C_6$)alkoxy group, a linear or branched ($C_1$-$C_6$)trihaloalkyl group, a linear or branched ($C_1$-$C_6$)trihaloalkoxy group, or an amino group (optionally substituted by one or two linear or branched ($C_1$-$C_6$)alkyl groups, which may be identical or different, each independently of the other),

isomers thereof and addition salts thereof with a pharmaceutically acceptable acid or base, with the proviso that, when $R_1$ and $R_3$ simultaneously represent a hydrogen atom, $R_2$ cannot represent a phenyl group optionally substituted in the *para* position by a bromine atom, chlorine atom, fluorine atom, methoxy group or hydroxy group.

**2.** Compounds of formula (I) according to claim 1, **characterised in that** $R_2$ represents a phenyl group substituted by at least one group selected identically or differently from halogen, linear or branched ($C_1$-$C_6$)alkyl, nitro, cyano, hydroxy, linear or branched ($C_1$-$C_6$)alkoxy, linear or branched ($C_1$-$C_6$)trihaloalkoxy, amino, mono- or di-($C_1$-$C_6$) alkylamino in which each alkyl group may be linear or branched, linear or branched ($C_1$-$C_6$)acyl, carboxy, linear or branched ($C_1$-$C_6$)alkoxycarbonyl, methylenedioxy, ethylenedioxy, linear or branched ($C_1$-$C_6$)alkylcarbonyloxy, and aryl-($C_1$-$C_6$)alkoxy in which the alkoxy moiety may be linear or branched,
isomers thereof and addition salts thereof with a pharmaceutically acceptable acid or base.

**3.** Compounds of formula (I) according to either claim 1 or claim 2, **characterised in that** $R_2$ represents a phenyl group substituted by at least one group selected from hydroxy, linear or branched ($C_1$-$C_6$)alkoxy and linear or branched ($C_1$-$C_6$)alkylcarbonyloxy, isomers thereof and addition salts thereof with a pharmaceutically acceptable acid or base.

**4.** Compounds of formula (I) according to claim 1, **characterised in that** $R_1$ represents a hydrogen atom and $R_3$ represents a hydrogen atom, isomers thereof and addition salts thereof with a pharmaceutically acceptable acid or base.

**5.** Compound of formula (I) according to claim 1 which is 3-(3,4-dihydroxyphenyl)-8*H*-thieno[2,3-*b*]pyrrolizin-8-one.

**6.** Compound of formula (I) according to claim 1 which is 2-methoxy-5-(8-oxo-8*H*-thieno[2,3-*b*]pyrrolizin-3-yl)phenyl acetate.

**7.** Process for the preparation of compounds of formula (I), **characterised in that** there is used as starting material a compound of formula (II) :

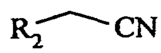

(II),

wherein $R_2$ is as defined for formula (I),
which compounds of formula (II) are reacted, in the presence of an alkali metal, with ethyl formate to yield the compounds of formula (III) :

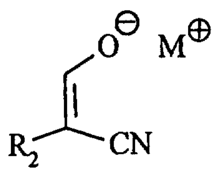

(III),

wherein $R_2$ is as defined hereinbefore and $M^+$ represents an alkali metal cation, such as the sodium or potassium cation,
which compounds of formula (III) are placed in conditions of nucleophilic addition in the presence of phenylsulphonyl chloride to yield the compounds of formula (IV) :

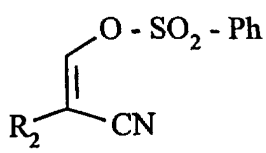

(IV),

wherein $R_2$ is as defined hereinbefore,
which compounds of formula (IV) are reacted with methyl thioglycolate to yield the compounds of formula (V) :

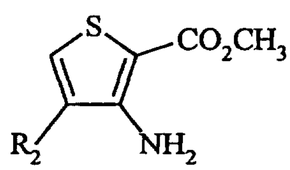

(V) ,

wherein $R_2$ is as defined hereinbefore,
the amino group of which compounds of formula (V) is protected according to conventional methods of organic synthesis and which are then reacted with a compound of formula (VI) :

$$R_3 - X \qquad (VI),$$

wherein X represents a leaving group and $R_3$ is as defined for formula (I),
the amino function of which is then deprotected to yield the compounds of formula (VII) :

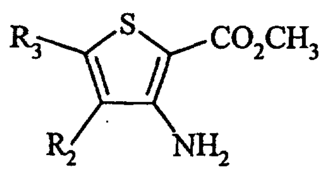

(VII) ,

wherein $R_2$ and $R_3$ are as defined for formula (I), which compounds of formula (VII) are placed in the presence of dimethoxytetrahydrofuran and 4-chloropyridinium chloride to yield the compounds of formula (VIII) :

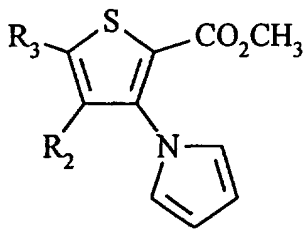

(VIII) ,

wherein $R_2$ and $R_3$ are as defined hereinbefore, which are then reacted with pyrrolidine to yield the compounds of formula (IX) :

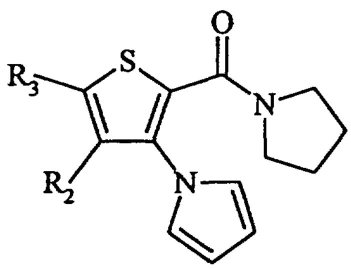

(IX) ,

wherein $R_2$ and $R_3$ are as defined hereinbefore, which compounds of formula (IX) are cyclised using phosphorus oxychloride to yield the compounds of formula (I/a), a particular case of the compounds of formula (I) :

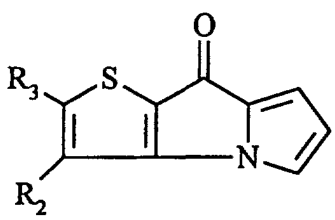

(I/a),

wherein $R_2$ and $R_3$ are as defined hereinbefore, the pyrrole nucleus of which compounds of formula (I/a) is functionalised according to conventional conditions of organic synthesis to yield the compounds of formula (I/b), a particular case of the compounds of formula (I) :

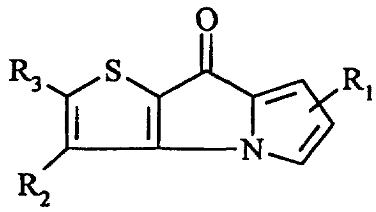

(I/b),

wherein $R_2$, $R_3$ and $R_1$ are as defined for formula (I) except that $R_1$ cannot represent a hydrogen atom, the compounds (I/a) and (I/b) constituting the totality of the compounds of the invention, which, if necessary, are purified according to a conventional purification technique, if desired, may be separated into their different isomers according to a conventional separation technique and, where appropriate, are converted into their addition salts with a pharmaceutically acceptable acid or base.

**8.** Pharmaceutical compositions comprising as active ingredient at least one compound of formula (I) according to any one of claims 1 to 6, alone or in combination with one or more pharmaceutically acceptable, inert, non-toxic excipients or carriers.

**9.** Pharmaceutical compositions according to claim 8 comprising at least one active ingredient according to any one of claims 1 to 6 for use in the treatment of cancer.